Europäisches Patentamt

(19) European Patent Office

Office européen des brevets

(11) Publication number: **0 088 602**
A2

# (12) EUROPEAN PATENT APPLICATION

(21) Application number: 83301166.1

(22) Date of filing: 04.03.83

(51) Int. Cl.³: **C 12 N 9/02**, C 12 P 1/00, C 12 P 7/02, C 12 P 7/24, C 12 P 17/02 // C12R1/00

(30) Priority: 08.03.82 US 355322
14.02.83 US 465823

(43) Date of publication of application: 14.09.83
Bulletin 83/37

(84) Designated Contracting States: DE FR GB

(71) Applicant: Exxon Research and Engineering Company, P.O.Box 390 180 Park Avenue, Florham Park New Jersey 07932 (US)

(72) Inventor: Patel, Ramesh Narsibhai, 15 Mockingbird Road, Edison New Jersey (US)
Inventor: Hou, Ching-Tsang, 14 Pendleton Place, Edison New Jersey (US)
Inventor: Laskin, Allen I., RD3, Box 392T, Somerset New Jersey (US)

(74) Representative: Field, Roger Norton et al, ESSO Engineering (Europe) Ltd. Patents & Licences Apex Tower High Street, New Malden Surrey KT3 4DJ (GB)

(54) Microbiological oxidation process.

(57) The soluble fractions of facultative organisms grown on $C_1$ compounds are capable of oxidizing organic compounds in the presence of a cofactor system comprissing $NADH_2$ or $NADPH_2$. The soluble fraction is obtained by aerobically growing the microorganism in a fermentor under continuous gassing with a mixture of a $C_1$ compound and air and, initially, carbon dioxide before harvesting. Preferably, the $C_1$ compound is methane.

One facultative organism which is useful in the microbiological oxidation of oxidizable organic substrates is Methylobacterium organophilum (CRL. 26, NRRL B-11, 222). This newly discovered and isolated methylotrophic microorganism strain and its natural and/or artificial mutants grow well under aerobic conditions in a culture medium in the presence of a $C_1$ compound as the major carbon and energy source.

ACTORUM AG

The present invention relates to a soluble fraction of a facultative organism which utilizes compounds containing one carbon atom which is capable of oxidizing a wide variety of organic compounds in the presence of a cofactor system of $NADH_2$ or $NADPH_2$. Among useful such organisms is a newly discovered and isolated methylotrophic microorganism strain which grows well under aerobic conditions in a culture medium in the presence of a $C_1$ compound, preferably methane, as the major carbon and energy source.

Methane-utilizing microorganisms are generally known as "methylotrophs". The classification system for methylotrophs proposed by R. Whittenbury et al. (J. of Gen. Microbiology, 61, 205-218 (1970)) is the most widely recognized. In their system, the morphological characteristics of methane-oxidizing bacteria are divided into five groups: Methylosinus, Methylocystis, Methylomonas, Methylobacter and Methylococcus.

Recently, Patt, Cole and Hanson (International J. Systematic Bacteriology, 26, (2) 226-229 (1976)) disclosed that methylotrophic bacteria are those bacteria that can grow non-autotrophically using carbon compounds containing one or more carbon atoms but containing no carbon-carbon bonds. Patt et al. have proposed that methylotrophs should be considered "obligate" if they are capable of utilizing only carbon compounds containing no carbon-carbon bonds (e.g., methane, methanol, dimethylether, methylamines, etc.) as the sole sources of carbon and energy whereas "facultative" methylotrophs are those organisms that can use both compounds containing no carbon-carbon bonds as well as compounds having carbon-carbon bonds as the sources of carbon and energy. In their paper, Patt et al.

disclosed a methane-oxidizing bacterium, which they identified as _Methylobacterium organophilum_ sp nov. (ATCC 27,886). This bacterium presumably differs from all previously described genera and species of methane-oxidizing bacteria because of its ability to utilize a variety of organic substrates with carbon-carbon bonds as sources of carbon and energy.

Hutchinson, Whittenbury and Dalton (_J. Theor. Biol._, _58_, 325-335 (1976)) and Colby and Dalton (_J. Biochem._, _157_, 495-497 (1976)) reported that ethylene is oxidized by the soluble methane monooxygenase from _Methylococcus capsulatus_ Strain Bath. The latter investigators reported that the "particulate membrane preparations" of _Methylococcus capsulatus_ Strain Bath did not have methane-oxygenase activity as determined by the bromomethane disappearance test.

Most recently, Stirling et al., _J. Biochem_, _96_, 205 (1979) and _J. Gen. Microbiol._, _116_, 277 (1980) reported that the obligate methane-utilizing methylotroph, _Methylosinus trichosporium_ OB3b, contained a soluble methane mono-oxygenase activity similar to that of the soluble methane monooxygenase from the _Methylococcus capsulatus_ Strain Bath. U.K. Patent No. 1,603,864 discloses a process for oxidation of selected organic substrates employing _Methylococcus capsulatus_ or _Methylosinus trichosporium_ as soluble fractions.

The present invention is directed to a soluble fraction of a facultative organism or genetically engineered derivative thereof or natural mutant thereof grown on a $C_1$ compound (i.e., a compound containing one carbon atom) which is characterized as being capable of oxidizing organic compounds in the presence of a

cofactor system comprising $NADH_2$ or $NADPH_2$. Examples of suitable oxidations of organic compounds to their oxidation products in accordance with this invention include converting alkanes to alcohols and methyl ketones, sec. alcohols to the corresponding methyl ketones, cyclic hydrocarbons to cyclic hydrocarbyl alcohols (e.g., cyclohexane to cyclohexanol), alkenes to 1,2-epoxides, styrene to styrene oxide, etc.

Two suitable organisms for this purpose are of the strains Methylobacterium organophilum (CRL.26) (NRRL B-11,222) and Methylobacterium organophilum (ATCC 27,886). While the latter is a known organism, the former strain is a newly discovered and isolated methylotrophic microorganism strain. This strain and its natural and/or artificial mutants grow well under aerobic conditions in a culture medium in the presence of a $C_1$ compound (methane or a methyl-radical-donating carbon-containing compound) as the major carbon and energy source.

The enzyme-active soluble fraction herein is obtained by growing the organism in a shake flask, followed by growth in a fermentor under continuous gassing with a mixture of a $C_1$ compound and air, disintegrating the cell suspension obtained thereby, and centrifuging the disintegrated cell suspension. The supernatant solution (representing the soluble fraction) obtained from the centrifugation may be used to oxidize secondary alcohols to ketones in the presence of a cofactor system comprising $NADH_2$ or $NADPH_2$. This is in contrast to the enzyme-active particulate fraction which is ordinarily obtained by growing the organism in a shake flask and harvesting the resultant organism.

The term "organism" is used herein to include bacteria, yeast, fungi, etc., preferably bacteria, capable of oxidizing methane and methyl-radical-donating carbon-containing compounds. The term "facultative" refers to organisms which can use both compounds containing no carbon-carbon bonds as well as compounds having carbon-carbon bonds as the sources of carbon and energy.

The term "genetically engineered derivatives" is used herein in the sense recognized by those skilled in the art, and includes artificial mutants of the organism and recombinant DNA-produced organisms such as may be produced from the plasmid DNA contained in Methylobacterium organophilum as reported by P.J. Warner et al., FEMS Microbiol. Lett., 1, 339 (1977).

The term "soluble fraction" refers to the enzyme activity in the supernatant solution obtained on harvesting the organism after growth in a fermentor (subsequent to growth in the shake flask) under continuous gassing with a mixture of a $C_1$ compound and air, when the broken cells are centrifuged at no less than 10,000 x g. for at least 15 minutes.

The term "increasing the oxidative state of an oxidizable organic compound" is meant to include incorporating oxygen in an organic compound, such as in epoxidizing olefins and converting alkanes to alcohols or ketones or increasing the oxidative state of oxygen-containing organic compounds such as converting alcohols to aldehydes and ketones (i.e., a dehydrogenating reaction). The soluble fraction obtained from methane-grown microbial cells is preferably used to oxidize alkenes to the corresponding epoxides and alcohols, alkanes to the corresponding alcohols and ketones, ethers to the corresponding alcohols and aldehydes, benzene to phenol, and carbon monoxide to carbon dioxide.

The expression "cofactor system comprising $NADH_2$ or $NADPH_2$" as used herein refers to a system comprised of $NADH_2$ or $NADPH_2$ or equivalents thereof, i.e., systems which will (re)generate $NADH_2$ or $NADPH_2$ in the oxidation process. Thus, the above expression includes a cofactor system comprising $NAD^+$, a substrate and a $NAD^+$-linked dehydrogenase for the substrate, which system will regenerate $NADH_2$ in situ.

The classification system of methane-oxidizing bacteria proposed by R. Whittenbury, K. C. Phillips and J. F. Wilkinson [J. Gen. Microbiology, 61, 205-218 (1970) (hereinafter Whittenbury et al.)] is the most widely recognized system used today. In this system of classification, based on morphological characteristics, methane-utilizing bacteria are divided into five groups. They are: Methylosinus, Methylocystis, Methylomonas, Methylobacter and Methylococcus. Bacteria of these five groups reported by Whittenbury et al. utilize methane, dimethyl ether, and methanol for growth energy and they were all reported as strictly aerobic and gram-negative.

As one embodiment of the present invention, we have discovered and isolated a new facultative strain identified below which grows well in a culture medium in the presence of oxygen and $C_1$ compounds (i.e., methane and methyl-radical-donating compounds) such as methanol, methylamine, methyl formate, methyl carbonate, dimethyl ether, etc. The preferred $C_1$ compounds herein are methane and methanol, most preferably methane. This newly discovered and isolated methylotrophic microorganism strain is capable of producing microbial cells useful as feedstuffs when cultured under aerobic conditions in a liquid growth medium comprising assimilable sources of nitrogen and essential mineral salts in the presence of methane gas or the above-mentioned methyl-radical-donating carbon-containing compounds as the major carbon and energy source.

As another embodiment of the invention there is provided a soluble fraction of a facultative organism or genetically engineered derivative thereof or natural mutant thereof, including the new strain mentioned above, which is capable of producing microbial cells when cultivated in an aerobic nutrient medium containing methane or the above-mentioned methyl-radical-donating carbon-containing compounds as the major carbon and energy source. The soluble fraction is obtained by aerobically growing the organism in a fermentor under continuous gassing with a mixture of a $C_1$ compound and air.

As still another embodiment of the invention there is provided a process for increasing the oxidative state of an oxidizable organic compound which comprises contacting, under aerobic conditions, in a medium comprising assimilable sources of nitrogen and essential mineral salts, the soluble fraction described above and the organic compound until at least a portion of the corresponding oxidized product is produced in isolable amounts, wherein the organism has been cultured, under aerobic conditions, in a fermentor under continuous gassing with a mixture of a $C_1$ compound (preferably methane) and air, and initially carbon dioxide.

A particularly preferred embodiment of the invention includes a process for producing propylene oxide from propylene by contacting propylene under aerobic conditions with the soluble fraction of a facultative organism which has been previously grown under aerobic conditions in the presence of methane.

The present invention includes the following features:

- The isolates of $C_1$-utilizing microbes of the invention are facultative organisms.

- If the microorganism is grown in a fermentor under continuous gassing with a mixture containing methane and air, and initially carbon dioxide, after growth in a shake flask, the soluble fraction of the organism, which will oxidize substrates in the presence of a cofactor system of $NADH_2$ or $NAPDH_2$, will be obtained. After cell growth, the cell suspension is disintegrated and centrifuged.

- If grown on methane, the soluble fraction converts alkanes to alcohols and ketones, alkenes to epoxides and alcohols, ethers to alcohols, benzene to phenol, toluene to benzyl alcohol and cresol, and carbon monoxide to carbon dioxide.

- The catalysts using the soluble fraction are not inhibited by such potential inhibitors as metal-binding or metal-chelating agents such as $\alpha,\alpha$-bipyridyl, thiosemicarbazide, thiourea, potassium cyanide, imidazole and 1,10-phenanthroline. Sulfhydryl agents such as iodoacetamide and 5',5'-dithiobis-2-nitrobenzoate and acriflavin inhibit monooxygenase activity.

The facultative organisms which may be employed in the present invention must utilize $C_1$ compounds. Preferably, the organisms are of the genus _Methylobacterium_, and more preferably they are of the species Methylobacterium organophilum. According to the classification system described in Bergey's Manual of Determinative Bacteriology, Robert S. Breed et al., eds., 8th ed., (Baltimore: Williams & Wilkins Co., 1974), this species has the following taxonomical and morphological characteristics: it produces colonies on salt agar plates in the presence of methane or methanol. The organisms are motile, rod-shaped, gram-negative, aerobic and grow at the expense of methane, methanol, glucose, succinate and nutrient agar. (Therefore, it is classically a facultative type.) It has a Type II membrane structure.

A newly discovered and isolated methane and methyl-radical-utilizing (methylotrophic) facultative organism strain useful in the present invention has the following identifying characteristics:

| Methylotrophic Organism Strain Name | ER&E Designation | U.S.D.A. Agriculture Research Center Designation |
|---|---|---|
| Methylobacterium organophilum | (CRL 26 R6) [hereinafter (CRL.26)] | NRRL B-11,222 |

An important characteristic of this strain is its capability to produce microbial cells (white colonies in this case) when cultured under aerobic conditions in a liquid growth medium comprising assimilable sources of nitrogen and essential mineral salts in the presence of methane gas or a methyl-radical donating carbon-containing compound such as methanol, methylamine, methyl formate, methyl carbonate, dimethyl ether, etc. as the major carbon and energy source.

The above strain has been deposited at the United States Department of Agriculture, Agriculture Research Service, Northern Regional Research Laboratory (NRRL), Peoria, Illinois 61604 and has received from NRRL the individual NRRL designation as indicated above pursuant to a contract between NRRL and the assignee of this patent application (Exxon Research and Engineering Company (ER&E)). The contract with NRRL provides for permanent availability of the progeny of this strain to the international public. This strain has been published in U.S. patent 4266034 and GB 2018822A. The assignee of the present application has agreed that, if this strain on deposit should die, or is destroyed, during the effective life of the patent, it will be replaced with a living strain of the same organism, It should be understood, however, that the availability of a deposit does not constitute a licence to practise the subject invention in derogation of patent rights granted by governmental action.

The newly discovered and isolated strain of the present invention was obtained from soil samples which were screened for methylotrophic microorganisms by growth under oxygen and methane. The methylotroph was then isolated, purified, and maintained by the procedure described below.

Another facultative organism strain which may be used in the present invention is Methylobacterium organophilum having ATCC designation 27,886. This strain produces pink colonies when cultured on salt agar plates in the presence of methane or methanol. Subcultures of the strain were deposited with the depository of the American Type Culture Collection (ATCC) in Rockville, Maryland 20852. The strain is more fully described by T. E. Patt et al., J. Bacteriol., 120, 955 (1974).

The maintenance of the cultures of the organisms for use in the present invention should be carefully controlled. The preferred means for maintaining the cultures is described below in Table II.

## Table II
### MAINTENANCE OF CULTURES

The organism is preferably subcultured every two weeks on mineral salts agar plates which contain medium having the following composition:

| | |
|---|---|
| $Na_2HPO_4$ | 0.21 g |
| $NaH_2PO_4$ | 0.09 g |
| $NaNO_3$ | 2.0 g |
| $MgSO_4 \cdot 7H_2O$ | 0.2 g |
| KCl | 0.04 g |
| $CaCl_2$ | 0.015 g |
| $FeSO_4 \cdot 7H_2O$ | 1 mg |
| $CuSO_4 \cdot 5H_2O$ | 0.01 mg |
| $H_3BO_4$ | 0.02 mg |
| $MnSO_4 \cdot 5H_2O$ | 0.14 mg |
| $ZnSO_4$ | 0.02 mg |
| $MoO_3$ | 0.02 mg |
| Agar | 15 g |
| Water | 1 liter |

These plates should be incubated in glass dessicators which have lids with an airtight seal and external sleeves with a tooled hose connection. Dessicators are to be evacuated and filled with a gas mixture

of a $C_1$ compound, preferably methane, and air (1:1 v/v). Incubation should be at 30°C. Cultures will survive in these dessicators for three months at 4°C. However, frequent transfer of cultures is preferred.

In commercial processes for the propagation of microorganisms, it is generally necessary to proceed by stages. These stages may be few or many, depending on the nature of the process. Ordinarily, propagation is started by inoculating cells from a slant of a culture into a pre-sterilized nutrient medium usually contained in a shake flask. In the flask, growth of the microorganisms is encouraged by various means, e.g., shaking for thorough aeration, and maintenance of suitable temperature. This step or stage is repeated one or more times in flasks or vessels containing the same or larger volumes of nutrient medium. These stages may be conveniently referred to as culture development stages. The microorganism with or without accompanying culture medium, from the last development stage, may be introduced or inoculated into a large-scale fermentor to produce commercial quantities of the microorganism or enzymes therefrom.

Reasons for growing the microorganism in stages are manifold, but are primarily dependent upon the conditions necessary for the growth of the microorganism and/or the production of enzymes therefrom. These include stability of the microorganism, proper nutrients, pH, osmotic relationships, degree of aeration, temperature and the maintenance of pure culture conditions during fermentation. For instance, to obtain maxiumum yields of the microbial cells, the conditions of fermentation in the final stage may have to be changed somewhat from those practised to obtain growth of the microorganisms in the culture development stages. Maintaining the purity of the medium, also, is an extremely important consideration, especially where the fermentation is performed under aerobic conditions as in the case of the methylotroph microorganisms. If the fermentation is initially started in a large fermentor, a relatively long period of time will be needed to achieve an appreciable yield of microorganisms and/or oxidative and dehydrogenase enzymes therefrom. This, of course, enhances the possibility of contamination of the medium and mutation of the microorganism.

The culture media used for growing the methylotrophic microorganism and inducing the oxidative enzyme system will be comprised of inorganic salts of phosphate, sulfates and nitrates as well as oxygen and a source of $C_1$ compounds. The fermentation will generally be conducted at temperatures ranging from 5 to about 50°C, preferably at temperatures ranging from about 25 to about 45°C. The pH of the culture medium should be controlled at a pH ranging from about 4 to 9 and preferably from about 5.5 to 8.5 and more preferably from 6.0 to 7.5. The fermentation may be conducted at atmospheric pressures although higher pressures up to about 5 atmospheres and higher may be employed.

Typically, to grow the methylotrophic microorganism and to induce the oxygenase and dehydrogenase enzymes, the microorganism is inoculated into the medium which is contacted with a gas mixture containing methane and oxygen. Methane may be supplied in the form of natural gas. For continuous flow culture the microorganisms may be grown in any suitably adapted fermentation vessel, for example, a stirred baffled fermentor or sparged tower fermentor, which is provided either with internal cooling or an external recycle cooling loop. Fresh medium may be continuously pumped into the culture at rates equivalent to 0.02 to 1 culture volume per hour and the culture may be removed at a rate such that the volume of culture remains constant. A gas mixture containing methane and oxygen and possibly carbon dioxide or other gases is contacted with the medium preferably by bubbling continuously through a sparger at the base of the vessel. The source of oxygen for the culture may be air, oxygen or oxygen-enriched air. Spent gas may be removed from the head of the vessel. The spent gas may be recycled either through an external loop or internally by means of a gas inducer impeller. The gas flows and recycle should be arranged to give maximum utilization of methane.

The soluble fraction of the organism having enzyme activity is obtained by further culturing the organism, after growth in a shake flask, in a fermentor under specified conditions before harvesting thereof. Thus, a sterile liquid culture medium as described above which is charged to a fermentor is inoculated with the bacteria grown in the shake flask. The inoculated mixture is stirred while a continuous constant stream of filtered air and a $C_1$ compound, and initially carbon dioxide, is allowed to flow through the fermentor. The $C_1$ compound may be methane or any methyl-radical donating carbon-containing compound such as, e.g., methanol, methylamine, methyl formate, methyl carbonate, dimethyl ether or the like, but is preferably methane. In general, the ratio of $C_1$ compound to air, by volume, in the gaseous mixture used for growing the cells is preferably no greater than about 1:2, and most preferably between 1:6 and 1:8, to avoid unnecessary excesses of methane. The pH of the growth medium in the fermentor is maintained in the range of 6 to 9, preferably 6 to 8, and most preferably 6 to 7, to obtain a satisfactory rate of cell growth. When these conditions are maintained, the soluble fraction rather than the particulate fraction will contain the enzyme activity.

After sufficient growth of the cells, the organism is harvested. In a typical procedure the contents of the fermentor are removed and centrifuged to remove the water therefrom. The residual cellular solid is suspended in a buffer solution (pH about 6 to 9), washed, centrifuged and resuspended in the buffer solution. The cell suspension thus obtained is then disintegrated so that the cells are broken down. This is ordinarily and preferably accomplished in a French pressure cell, into which the cell suspension is injected. The pressure cell, which is a block of steel with a piston and a chamber for the suspension, exerts a high pressure (e.g., up to 60 mPa or greater) on the suspension, and when the cell is opened, the rapid release of pressure causes the cells to disintegrate.

The disintegrated cells are separated into a particulate fraction and a supernatant solution by centrifuging the cell suspension at a force of at least 10,000 x g. for at least 15 minutes. The supernatant solution represents the soluble fraction useful in the oxidation of various organic substrates. In a preferred embodiment, the separation step is carried out by at least two centrifugation operations wherein the supernatant solution from the first operation is centrifuged at a greater centrifugation force than was used for the first centrifugation. Especially preferred is isolation of the soluble fraction by two or a series of successive centrifugations at successively greater centrifugation forces up to a maximum force representing the mechanical limitation of the machine.

The enzyme-active soluble fraction is then brought into contact with the desired oxidizable organic substrate, e.g., a $C_2$-$C_4$ alkene, e.g., ethylene, propylene, butene-1 or conjugated butadiene or mixtures thereof, a cyclic compound such as cyclohexane, an alkane such as methane, ethane, propane or butane, etc., or a secondary alcohol, e.g., 2-propanol or 2-butanol in the presence of oxygen and a buffer solution or nutrient medium (e.g., the same nutrient medium used to produce the microorganism may be used except that the oxidizable substrate material has replaced the methane) and the mixture is incubated until the desired degree of conversion has been obtained. Thereafter, the oxidized product is recovered by conventional means, e.g., distillation, etc.

The soluble fraction may be used to catalyze the oxidation of several oxidizable organic compounds, including oxidation of alkenes to the corresponding epoxides, e.g., ethylene to ethylene oxide, propylene to propylene oxide, 1-butene to 1,2-epoxybutane, butadiene to 1,2-epoxybutene, isobutène to epoxyisobutane, cis-but-2-ene to cis-2,3-epoxybutane and cis-2-buten-1-ol, trans-but-2-ene to trans-2,3-epoxybutane, etc., preferably, linear, branched, substituted, terminal or internal olefins. The soluble fraction also promotes oxidation of linear and branched alkanes to the corresponding primary, secondary or tertiary alcohols, such as, e.g., methane to methanol, ethane to ethanol, propane to 1-propanol and 2-propanol, butane to 1-butanol and 2-butanol, pentane to 1-pentanol and 2-pentanol, hexane to 1-hexanol and 2-hexanol, heptane to 1-heptanol and 2-heptanol, octane to 1-octanol and 2-octanol, isobutane to isobutanol and tert-butanol, cyclohexane to cyclohexanol, toluene to benzyl alcohol and cresol, etc., preferably linear, branched, cyclic or aryl alkanes. Additional oxidation reactions include oxidation of ethers to the corresponding alcohols and aldehydes such as, e.g., dimethylether to methanol and formaldehyde; substituted alkanes to aldehydes such as, e.g., chloro-, bromo-, or fluoromethanes to formaldehyde, oxidized dihalomethanes, and oxidized trihalomethanes;

esters to the corresponding aldehydes, such as, e.g., methylformate to formaldehyde; benzene to phenol; and carbon monoxide to carbon dioxide.

The oxidation reactions using the soluble fraction must take place under aerobic conditions in the presence of a cofactor system comprising nicotinamide adenine dinucleotide in the reduced form ($NADH_2$) or nicotinamide adenine dinucleotide phosphate in the reduced form ($NADPH_2$). The cofactor which is initially present in the cell fraction is ordinarily removed therefrom during the purification process and must be replenished to effect oxidation using the soluble fraction. The pH of the oxidation reaction using the soluble fraction may range from 6 to 9, depending mainly on the substrate used, preferably 6-8, most preferably 6-7, and the temperature may range from about 20-80°C, preferably 30-50°C, depending mainly on the substrate employed.

The $NADH_2$ cofactor system herein may be prepared by adding $NADH_2$ exogenously to the oxidation reaction mixture containing the soluble fraction or it may be generated (and/or regenerated) in situ. In the latter case, an $NAD^+$-linked dehydrogenase enzyme and its substrate may be used in the presence of $NAD^+$ to produce $NADH_2$ as election donor for the enzyme. Examples of preferred cofactor systems for (re)generation of $NADH_2$ include a system of formate and $NAD^+$ (with the $NAD^+$-linked formate dehydrogenase present in the soluble fraction), a system of formaldehyde, $NAD^+$ and formaldehyde dehydrogenase, or a system of a secondary alcohol, such as 2-butanol, $NAD^+$, and an $NAD^+$-linked secondary alcohol dehydrogenase. The latter three systems are found to increase the rate of reaction two to eight fold over the rate when $NADH_2$ is added exogenously.

To facilitate the necessary effective contact of oxygen and the enzyme, it is preferred, for best results, to introduce a strong, finely divided air stream into a vigorously stirred dispersion of substrate in the oxidation medium that generally contains water, and a buffer in which the enzyme preparation or microorganism culture is suspended. The enzyme preparation may then be separated from the liquid medium, preferably by filtration or centrifugation. The resulting oxidized product may then generally be obtained.

The process of the invention may be carried out batchwise, semicontinuously, continuously, concurrently or countercurrently. Optionally, the suspension containing the enzyme preparation or methylotrophic microorganism and buffer solution is passed downwardly with vigorous stirring countercurrently to an air stream rising in a tube reactor. The top layer is removed from the downflowing suspension, while culture and remaining buffer solution constituents are recycled, at least partly, with more oxidative substrate and addition of fresh enzyme preparation or methylotrophic microorganism, as required.

The growth of the methylotrophic microorganism and the oxidation process may be conveniently coupled by conducting them simultaneously, but separately and using much higher aeration in the oxidation process (e.g., an air excess of at least twice that required for growth, preferably at least five times as much aeration). Both the growth process and the methane hydroxylation or oxidation processes may be conducted in the same reactor in sequential or simultaneous operations by alternate use of normal and strong aeration.

The oxidation reaction should not be carried out in the presence of a substrate competing for the same enzyme system, and thus, none of the oxidation reactions should be carried out in the presence of methane except, of course, when methane is the substrate being oxidized to methanol.

The invention is illustrated further by the following examples which, however, are not to be taken as limiting in any respect. All parts and percentages, unless expressly stated otherwise, are by weight.

EXAMPLE 1 - Preparation of Soluble Fraction of Methane Monooxygenase From Facultative Methylobacterium Organophilum (CRL.26)

The facultative methane-utilizing organism, Methylobacterium organophilum (CRL.26), was isolated from soil samples by enrichment culture using methane (methane and air, 50:50 parts by volume) as a carbon source, as described in Patel et al., J. Bacteriol, 136, 352 (1978). The organisms were maintained on mineral salts agar plates in a dessicator under an atmosphere of 1:1 by volume of methane: air at 30°C.

The organisms were grown on a small scale at 30°C in 2.8 $\ell$ flasks containing 800 ml of mineral salts medium with methane (1:1 parts by volume methane:air) as the sole carbon and energy source. Cells were harvested after 24-28 hours by centrifugation at 10,000 x g. for 15 min. Large scale cultures were grown on methane (10% methane, 15% carbon dioxide and 75% air) at 30°C in batch culture on a mineral salt medium in a 30-$\ell$ explosion-resistant fermentor. The fermentor was inoculated with 2$\ell$ of a culture grown in flasks.

The cells were washed twice with 25 millimolar potassium phosphate buffer at pH 7.0 and suspended in 25 millimolar potassium phosphate buffer at pH 7.0

containing 5 millimolar $MgCl_2$ and deoxyribonuclease (0.05 mg/ml). Cell suspensions at $4^{o}C$ were disintegrated by a single passage through a French pressure cell (American Instruments Co., Silver Spring, Md) at 60 mPa. Disintegrated cell suspensions were centrifuged at 15,000 x g. for 15 min. to remove unbroken cells. The supernatant solution was then centrifuged at 40,000 x g. for 60 min., yielding particulate pallet P(40) and soluble S(40) fractions. The soluble fraction was subsequently centrifuged at 80,000 x g. for 60 min., yielding particulate P(80) and soluble S(80) fractions.

EXAMPLE 2 - Hydroxylation of n-Alkanes

Several 3.0 ml vials at $4^{o}C$ were filled with 0.5 ml of a reaction mixture consisting of 25 micromoles potassium phosphate buffer at pH 7.0, 10 micromoles $NADH_2$, and the soluble S(80) fraction obtained as described in Example 1 or Methylococcus capsulatus Strain Bath as a comparison.

The vials were incubated at $35^{o}C$ on a reciprocating water bath shaker at 50 oscillations per minute. The gaseous phase of the vials was evacuated by vacuum and replaced with a 1 to 1 by volume gaseous mixture of the alkane substrate indicated in Table III to oxygen, at which point the reaction was initiated.

The rate of oxidation of the alkanes was measured by injecting 1-2 $\mu$ 1 samples of the reaction mixture into a gas chromatograph immediately after addition of substrate (zero time) and after 5 and 10 min. of incubation. Specific activities were expressed as nmoles of product formed per min. per mg. of protein, with the higher number representing better conversion. With each substrate, control experiments were conducted in the absence of $NADH_2$, in the absence of oxygen, and using boiled extracts.

The alcohol products were identified and estimated by retention time comparisons and co-chromatography with authentic standards using flame-ionization gas chromatography. The column temperature was maintained isothermally between 80°C and 200°C with helium carrier gas flow rates of 20-40 ml per min. The amount of product formed was estimated from peak areas using a standard graph constructed using authentic compounds.

Duplicate measurements were carried out for each substrate. Protein concentrations in cellular fractions were estimated with Folin Ciocalteu reagent as described by O.H. Lowry et al., J. Biol. Chem., 193, 265 (1951), using bovine serum albumin as a standard.

TABLE III

Hydroxylation of n-Alkanes

| Substrate | Product | Specific Activity (nmoles/min/mg of protein) | |
|---|---|---|---|
| | | CRL.26 | Bath strain* |
| Methane | Methanol | 93 | 84 |
| Ethane | Ethanol | 64 | 68 |
| Propane | 1-Propanol 2-Propanol | 37 | 69 |
| Butane | 1-Butanol 2-Butanol | 68 | 77 |
| Pentane | 1-Pentanol 2-Pentanol | 66 | 73 |
| Hexane | 1-Hexanol 2-Hexanol | 60 | 40 |
| Heptane | 1-Heptanol 2-Heptanol | 62 | 27 |
| Octane | 1-Octanol 2-Octanol | 19.5 | 9 |

* Comparative

EXAMPLE 3 - Oxidation of Substituted Alkane Derivatives

The procedure of Example 2 was followed using the substituted alkane derivatives in Table IV except that the rate of oxidation was measured by following the utilization of substrate from the gas or liquid phase. Thus, 2 $\mu$l samples of liquid or 50 $\mu$l samples of gas were injected into the gas chromatograph at zero time and after 5 min. and after 10 min. of incubation of the reaction mixture at 35°C. Specific activities were expressed as the amount of substrate utilized per minute per mg of protein in the S(80) fractions. Controls were used as described in Example 2. Detection of formaldehyde (the oxidation product of chloromethane, bromomethane and fluoromethane) was estimated colorimetrically by the Hantzsch reaction described by T. Nash, Biochem. J., 55, 416 (1953). The results are indicated in Table IV.

TABLE IV

Oxidation of Substituted Alkane Derivatives

| Substrate | Product | Specific Activity (nmoles/min/mg of protein) | |
|---|---|---|---|
| | | CRL.26 | Bath Strain* |
| Chloromethane | Formaldehyde | 44 | 84 |
| Bromomethane | Formaldehyde | 48 | 66 |
| Fluoromethane | Formaldehyde | 19 | NR |
| Dichloromethane | ND | 40 | 82 |
| Trichloromethane | ND | 21 | 35 |
| Nitromethane | ND | 12 | 45 |
| Nitroethane | ND | 18 | NR |
| 1-Nitropropane | ND | 50 | NR |
| 2-Nitropropane | ND | 19 | NR |
| 1-Bromobutane | ND | 49 | NR |
| 2-Bromobutane | ND | 12 | NR |
| Isobutane | Isobutanol Tert.-butanol | 74 | 17.6 |

*Comparative

ND: Product was not identified.

NR: Experiment was not run.

EXAMPLE 4 - Epoxidation of Alkenes

The procedure of Example 3 was followed to oxidize several alkenes, and the results are shown in Table V.

TABLE V

Epoxidation of Alkenes

| Substrate | Product | Specific Activity (nmoles/min/mg of protein | |
|---|---|---|---|
| | | CRL.26 | Bath Strain* |
| Ethylene | Ethylene Oxide | 55 | 148 |
| Propylene | Propylene Oxide | 100 | 83 |
| But-1-ene | 1,2-Epoxybutane | 87 | 49 |
| Butadiene | 1,2-Epoxybutene | 75 | NR |
| Isobutylene | 1,2-Epoxyisobutene | 95 | NR |
| Cis-but-2-ene | Cis-2,3-Epoxybutane Cis-2,Buten-1-ol | 37 | 141 |
| Trans-But-2-ene | Trans-2,3-Epoxybutane Trans-2-Buten-1-ol | 43 | 57 |
| 2-Methyl-1-butene | ND | 42 | NR |
| 2-Methyl-2-butene | ND | 16 | NR |
| 1-Bromo-1-butene | ND | 83 | NR |
| 2-Bromo-2-butene | ND | 30 | NR |
| Isoprene | 1,2-Epoxyisoprene | 38 | NR |

* Comparative

ND: Product was not identified.

NR: Experiment was not run.

It is seen that there are many differences in oxidation rates of alkanes, substituted alkanes, and alkenes when the soluble fraction containing methane monooxygenase from facultative methylotroph Methylobacterium organophilum (CRL.26) is employed rather than the soluble methane monooxygenase fraction from obligate methylotroph Methylococcus capsulatus Strain bath.

- 24 -

EXAMPLE 5 - Oxidation of Ethers and Carbon Monoxide

The procedure of Example 2 was followed to oxidize ethers and carbon monoxide, with the results indicated in Table VI.

TABLE VI

Oxidation of Ethers

| Substrate | Product | Specific Activity (nmoles/min/mg of protein) |
|---|---|---|
| Dimethylether | Methanol | 25 |
| | Formaldehyde | 10 |
| Butylether | ND | 34 |
| Carbon Monoxide | Carbon Dioxide | 30 |

ND: Product was not identified.

EXAMPLE 6 - Oxidation of Cycloalkyl and Aromatic Compounds

The procedure of Example 2 was followed to oxidize cyclohexane, benzene, and toluene, and the results are indicated in Table VII.

TABLE VII

Oxidation of Cyclic and Aromatic Compounds

| Substrate | Product | Specific Activity (nmoles/min/mg of protein) |
|---|---|---|
| Cyclohexane | Cyclohexanol | 36 |
| Toluene | Benzylalcohol | 22 |
| | Cresol | 15 |
| Benzene | Phenol | 20 |

EXAMPLE 7 - Effective Cofactor Systems

The procedure of Example 2 was followed using propylene as substrate except that various cofactor systems were used to replace $NADH_2$. The various cofactor systems, concentration thereof, and rates of conversion are indicated in Table VIII. As can be seen, only $NADH_2$ and $NADPH_2$ were suitable as electron donors. The remainder of electron donors, many known to be replacements for $NADH_2$ in other particulate and soluble methane monooxygenase systems, were ineffective. For example, formaldehyde in the absence of $NAD^+$ could act as an electron donor for the soluble methane monooxygenase fraction of the obligate methylotroph Methylococcus capsulatus Strain Bath.

## TABLE VIII

### Effect of Various Electron Donor Systems on Soluble Methane Monooxygenase

| Electron Donor | Concentration (mM) | Specific Activity (nmoles/min/mg of protein) |
|---|---|---|
| $NADH_2$ | 2.5 | 100 |
| $NADPH_2$ | 2.5 | 70 |
| Sodium-L-Ascorbate | 5.50 | 0 |
| Methanol | 5.50 | 0 |
| Methanol + Methanol Dehydrogenase | 5 + 100 $\mu$ g | 0 |
| Formaldehyde | 2.5 | 0 |
| Formaldehyde + $NAD^+$ | 2.5 + 2.5 | 0 |
| Formaldehyde + $NADP^+$ | 2.5 + 2.5 | 0 |
| Formate | 5 | 0 |

EXAMPLE 8 - Effect of Temperature and pH

The procedure of Example 2 was followed using propylene as a substrate but varying the pH between 6 and 9 and then varying the temperature from 20 to 45°C. The rates of conversion under these varied conditions are indicated in Table IX.

## TABLE IX

| pH | Temperature ($^{\circ}$C) | Activity (%)* |
|---|---|---|
| 6.0 | 40 | 70 |
| 6.5 | 40 | 85 |
| 7.0 | 40 | 100 |
| 7.5 | 40 | 90 |
| 8.0 | 40 | 80 |
| 8.5 | 40 | 65 |
| 9.0 | 40 | 60 |
| 7.0 | 20 | 18 |
| 7.0 | 25 | 36 |
| 7.0 | 30 | 60 |
| 7.0 | 35 | 90 |
| 7.0 | 40 | 100 |
| 7.0 | 45 | 76 |

*Activity is expressed as a percentage of the control (100%) represented by pH 7.0 and temperature of 40$^{\circ}$C.

EXAMPLE 9 - Effect of Potential Inhibitors

The procedure of Example 2 was followed except that a given concentration of a potential inhibitor (metal-binding or metal-chelating agent) given in Table X was incubated in the reaction mixture at 0$^{\circ}$C for 15 min. Reactions were initiated by gassing the vials with propylene. 8-Hydroxyquinoline, which strongly inhibits soluble methane monooxygenase from Methylococcus capsulatus (Bath) and Methylosinus trichosporium (OB3b), was not inhibitory to soluble methane monooxygenase from Methylobacterium organophilum (CRL.26). $\alpha,\alpha$Bipyridyl and 1,10-phenanthroline, which inhibited about 30% activity of Methylobacterium organophilum (CRL.26), did not inhibit the activity of Methylococcus capsulatus (Bath).

TABLE X

Effect of Metal-Binding and Sulfhydryl
Inhibitors on Soluble Methane Monooxygenase

| Inhibitor | Concentration (mM) | Activity (%)[a] | Inhibition (%)[a],[b] |
|---|---|---|---|
| Control | 1 | 100 | 0 |
| Thiosemicarbazide | 1 | 108 | 0 |
| 1,10-Phenanthroline | 1 | 67 | 33 |
| Potassium Cyanide | 1 | 108 | 0 |
| $\alpha,\alpha$-Bipyridyl | 1 | 74 | 24 |
| Thiourea | 1 | 100 | 0 |
| Imidazole | 1 | 130 | 0 |
| 8-Hydroxyquinoline | 1 | 117 | 0 |
| N-Ethylmaleimide | 1 | . 102 | 0 |
| Iodoacetamide | 1 | 20 | 80 |
| 5,5'-dithiobis-2-nitrobenzoate | 1 | 38 | 62 |
| Acriflavin | 0.1 | 71 | 29 |
| p-Hydroxymercuribenzoate | 0.1 | 100 | 0 |

(a) Activity and inhibition are expressed as a percentage of the control (100%) containing no inhibitor.

(b) The uninhibited rate of propylene oxidation was 100 nmoles of propylene oxide produced/min/mg of protein.

0088602

EXAMPLE 10 - Resolution of Soluble Fraction
Into Its Components

The soluble S(80) fraction (20 ml) from Example 1 was loaded on a diethylaminoethyl (DEAE) cellulose column (0.9 x 30 cm) equilibrated with 25mM potassium phosphate buffer at pH 7.0 containing 5mM $MgCl_2$ and 5mM dithiothreitol (buffer A). Proteins not adsorbed to DEAE-cellulose (fraction A) were eluted with buffer A. The column was then eluted with successive 25-ml batches of buffer A containing 0.2M NaCl, and 0.5M NaCl. Fractions from the 0.2M NaCl eluate (fraction B) having a dark brown color were combined. Fractions from the 0.5M NaCl eluate (fraction C) with a yellow color were also combined.

The specific activities of the S(80) fraction before resolution, of each fraction separately, of two combined fractions, and of the three combined fractions were measured as described in Example 2 for conversion of propylene to propylene oxide. The results are indicated in Table XI.

TABLE XI

Resolution of Soluble Methane Monooxygenase into
Three Fractions by DEAE-Cellulose Chromatography

| Fraction | Specific Activity (nmoles/min/mg of protein) |
|---|---|
| Before DEAE resolution | 95 |
| Fraction A | 0 |
| Fraction B | 0 |
| Fraction C | 0 |
| Fraction A + B (1:1) | 0 |
| Fraction A + C (1:1) | 65 |
| Fraction B + C (1:1) | 0 |
| Fraction A + B + C (1:1:1) | 125 |

It is seen that all three fractions are required for maximum activity in the oxidation, as is the case with <u>Methylococcus capsulatus</u> (Bath).

<u>EXAMPLE 11</u> - <u>Effective Cofactor Systems</u>

The procedure of Example 2 was followed using propylene as substrate except that various cofactor systems were used to replace $NADH_2$. The various cofactor systems, concentrations thereof, and rates of conversion are indicated in Table XII. As can be seen, of the systems tested, only $NADH_2$; $NADPH_2$; formate and $NAD^+$; formaldehyde, $NAD^+$ and formaldehyde dehydrogenase; and secondary butanol in the presence of secondary alcohol dehydrogenase (SADH) from yeast <u>Pichia</u> sp. and $NAD^+$ were suitable as cofactor systems. The remainder of the cofactor systems, many known to be electron donor replacements for $NADH_2$ in other particulate and soluble methane monooxygenase systems, were ineffective.

TABLE XII

Effect of Various Cofactor Systems on
Soluble Methane Monooxygenase

| Cofactor System | Concentration (mM) | Specific Activity (nmoles/min/mg of protein) |
|---|---|---|
| $NADH_2$ | 2.5 | 100 |
| $NADPH_2$ | 2.5 | 70 |
| Sodium-L-Ascorbate | 5.50 | 0 |
| Methanol | 5.50 | 0 |
| Methanol + Methanol Dehydrogenase | 5 + 100 $\mu$g | 0 |
| Formaldehyde | 2.5 | 0 |
| Formaldehyde + $NAD^+$ | 2.5 + 2.5 | 0 |
| Formaldehyde + $NADP^+$ | 2.5 + 2.5 | 0 |
| Formaldehyde + $NAD^+$ + Formaldehyde Dehydrogenase | 2.5 + 2.5 + 100 $\mu$g | 250 |
| Formate | 5 | 0 |
| Formate + $NAD^+$ | 5 + 2.5 | 240 |
| 2-Butanol + $NAD^+$ + Yeast Secondary Alcohol Dehydrogenase | 5 + 2.5 + 100 $\mu$g | 210 |

EXAMPLE 12 - Regeneration of $NADH_2$ Using $NAD^+$ with Formate or With Formaldehyde as Cofactor System

The procedure of Example 2 or 3 was followed using the alkane (Table XIII) or alkene (Table XIV) substrates indicated and a cofactor system of $NADH_2$ or formate + $NAD^+$. It can be seen that the rates of both the epoxidation and hydroxylation reactions increased two to four fold using $NAD^+$ and formate to regenerate $NADH_2$ rather than using $NADH_2$ itself as the electron donor.

TABLE XIII

Hydroxylation of Alkanes by Soluble Methane
Monooxygenase from Methylobacterium sp. (CRL.26)

| Substrate | Cofactor System | Product[a] | Specific Activity (nmoles/min/mg of protein) |
|-----------|-----------------|------------|----------------------------------------------|
| Methane | NADH$_2$ | Methanol | 62 |
| Methane | Formate + NAD$^+$ | Methanol | 125 |
| Ethane | NADH$_2$ | Ethanol | 64 |
| Ethane | Formate + NAD$^+$ | Ethanol | 147 |
| Propane | NADH$_2$ | 1-Propanol<br>2-Propanol | 15<br>22 |
| Propane | Formate + NAD$^+$ | 1-Propanol<br>2-Propanol | 62<br>112 |
| Butane | NADH$_2$ | 1-Butanol<br>2-Butanol | 43<br>25 |
| Butane | Formate + NAD$^+$ | 1-Butanol<br>2-Butanol | 344<br>200 |
| Pentane | NADH$_2$ | 1-Pentanol<br>2-Pentanol | 21<br>45 |

TABLE XIII (Cont'd)

| Substrate | Cofactor System | Product[a] | Specific Activity (nmoles/min/mg of protein) |
|---|---|---|---|
| Pentane | Formate + NAD$^+$ | 1-Pentanol | 84 |
| | | 2-Pentanol | 180 |
| Isobutane | NADH$_2$ | Isobutanol | 34 |
| | | Tert. Butanol | 40 |
| Isobutane | Formate + NAD$^+$ | Isobutanol | 78 |
| | | Tert. Butanol | 112 |
| 2-Bromobutane | NADH$_2$ | ND | 10 |
| 2-Bromobutane | Formate + NAD$^+$ | ND | 40 |
| Nitroethane | NADH$_2$ | ND | 18 |
| Nitroethane | Formate + NAD$^+$ | ND | 42 |

(a)    Products were identified and estimated by gas chromatography retention time comparison and co-chromatography with authentic standards.

ND =  Not determined.  Rate of oxidation was estimated by the rate of utilization of substrate.

TABLE XIV

Epoxidation of Alkenes by Soluble Methane
Monooxygenase from Methylobacterium sp. (CRL.26)

| Substrate | Cofactor System | Product(a) | Specific Activity (nmoles/min/mg of protein) |
|-----------|-----------------|------------|--------------------------------|
| Ethylene | $NADH_2$ | Ethylene Oxide | 55 |
| Ethylene | Formate + $NAD^+$ | Ethylene Oxide | 145 |
| Propylene | $NADH_2$ | Propylene Oxide | 89 |
| Propylene | Formate + $NAD^+$ | Propylene Oxide | 267 |
| 1-Butene | $NADH_2$ | 1,2-Epoxybutane | 87 |
| 1-Butene | Formate + $NAD^+$ | 1,2-Epoxybutane | 200 |
| Butadiene | $NADH_2$ | 1,2-Epoxybutene | 75 |
| Butadiene | Formate + $NAD^+$ | 1,2-Epoxybutene | 180 |
| Isobutene | $NADH_2$ | Epoxyisobutane | 95 |
| Isobutene | Formate + $NAD^+$ | Epoxyisobutane | 190 |

- 33 -

0088602

TABLE XIV (Cont'd)

| Substrate | Cofactor System | Product[a] | Specific Activity (nmoles/min/mg of protein) |
|---|---|---|---|
| 2-Methyl-1-Butene | $NADH_2$ | ND | 40 |
| 2-Methyl-1-Butene | Formate + $NAD^+$ | ND | 75 |

(a) Products were identified and estimated by gas chromatography retention time comparison and co-chromatography with authentic standards.

ND = Products were not identified. The rate of oxidation was measured by the rate of utilization of substrate.

The procedure of Example 2 was followed using a total of 0.5 ml of a reaction mixture containing 25 $\mu$moles potassium phosphate buffer at pH 7.0, 5 $\mu$moles NAD$^+$, 10 $\mu$moles formaldehyde, 5 $\mu$moles reduced glutathione, and, as the remainder, the soluble S(80) fraction obtained as described in Example 1, and purified formaldehyde dehydrogenase from a yeast, Pichia sp. The substrates employed, products produced, and rates of product formation are indicated in Table XV.

## TABLE XV

Regeneration of NAD$^+$/NADH$_2$:
Hydroxylation of Alkanes/Epoxidation of Alkenes by
Methane Monooxygenase from Methylobacterium sp.
CRL.26 and Oxidation of Formaldehyde

| Substrate | Product | Rate of Product Formation (nmoles/min/mg protein) [a] |
|---|---|---|
| Methane | Methanol | 128 |
| Ethane | Ethanol | 145 |
| Propane | Propan-1-ol | 60 |
| | Propan-2-ol | 95 |
| Butane | Butan-1-ol | 250 |
| | Butan-2-ol | 150 |
| Ethylene | Ethylene Oxide | 140 |
| Propylene | Propylene Oxide | 250 |
| 1-Butene | 1,2-Epoxybutane | 180 |

EXAMPLE 13 - Regeneration of $NADH_2$ Using $NAD^+$ With Secondary Alcohol Dehydrogenase as Cofactor System

The procedure of Example 2 was followed using the alkene (Table XVI) or alkane (Table XVII) substrates indicated and a cofactor system of $NADH_2$ alone, of a mixture of 2-butanol, $NAD^+$ and secondary alcohol dehydrogenase (SADH), or of a mixture of SADH and $NAD^+$. It can be seen that the rate of the epoxidation and hydroxylation reactions increased three to four fold using the mixture of $NAD^+$, SADH and 2-butanol to regenerate $NADH_2$ rather than using $NADH_2$ itself as the election donor.

EXAMPLE 14 - Preparation of Soluble Fraction of Methane Monooxygenase From Facultative Methylobacterium Organophilum (ATCC 27,886)

The facultative methane-utilizing organism, Methylobacterium organophilum (ATCC 27,886), was obtained from R. Hanson at the Gray Freshwater Biological Institute, University of Minnesota, Navarre, Minnesota. It was maintained on mineral salts agar plates in a dessicator under an atmosphere of 1:1 by volume of methane:air at 30°C.

The organisms were grown and harvested as described in Example 1. The soluble fraction was obtained as described in Example 1 except that after centrifugation at 40,000 x g. for 60 min. to yield the particulate P(40) and soluble S(40) fractions, the S(40) fraction was subsequently centrifuged at 80,000 x g. for 120 min., yielding particulate P(80) and soluble S(80) fractions.

EXAMPLE 15 - Hydroxylation of n-Alkanes

Several 3.0 ml vials at 4°C were filled with 0.5 ml of a reaction mixture consisting of 50 micromoles potassium phosphate buffer at pH 7.0, 10 micromoles $NADH_2$, and, as the remainder, the soluble S(80) fraction obtained as described in Example 14.

TABLE XVI

Oxidation of Alkenes by Soluble Methane
Monooxygenase From Methylobacterium sp. (CRL.26)

| Substrate + Cofactor System | Product[a] | Specific Activity[b] (nmoles/min/mg protein) |
|---|---|---|
| 1. Ethylene + NADH$_2$ | Ethylene Oxide | 55 |
| 2. Ethylene + 2-Butanol + SADH + NAD$^+$ | Ethylene Oxide 2-Butanone | 210 3000 |
| 3. Propylene + NADH$_2$ | Propylene Oxide | 93 |
| 4. Propylene + 2-Butanol + SADH + NAD$^+$ | Propylene Oxide 2-Butanone | 290 3000 |
| 5. 1-Butene + NADH$_2$ | 1,2-Epoxybutane | 87 |
| 6. 1-Butene + 2-Butanol + SADH + NAD$^+$ | 1,2-Epoxybutane 2-Butanone | 251 2800 |
| 7. Butadiene + NADH$_2$ | 1,2-Epoxybutene | 72 |
| 8. Butadiene + 2-Butanol + SADH + NAD$^+$ | 1,2-Epoxybutene 2-Butanone | 210 2800 |
| 9. Isobutene + NADH$_2$ | 1,2-Epoxyisobutane | 85 |
| 10. Isobutene + 2-Butanol + SADH + NAD$^+$ | 1,2-Epoxyisobutane 2-Butanone | 320 2800 |

TABLE XVI (Cont'd)

(a)  Products were identified and estimated by gas chromatography retention time comparison and co-chromatography with authentic standards.  Reactions were carried out at 35ºC on a shaker bath.  Partially purified secondary alcohol dehydrogenase (SADH) was prepared from a yeast, _Pichia_ sp.

(b)  Specific activities for epoxidation reactions using $NADH_2$ itself were expressed as the nmoles of product formed/min/mg. of protein in soluble fraction of _Methylobacterium_ sp. (CRL.26).  Specific activities for dehydrogenase reactions were expressed as the nmoles of 2-butanone formed/min/mg. of protein of purified yeast SADH.

TABLE XVII

Oxidation of Alkanes by Soluble Methane
Monooxygenase From Methylobacterium sp. (CRL.26)

| Substrate + Cofactor System | Products[a] | Specific Activity[b] (nmoles/min/mg protein) |
|---|---|---|
| 1. Methane + $NADH_2$ | Methanol | 87 |
| 2. Methane + 2-Butanol + SADH + $NAD^+$ | Methanol 2-Butanone | 251 2900 |
| 3. Ethane + $NADH_2$ | Ethanol | 64 |
| 4. Ethane + 2-Butanol + SADH + $NAD^+$ | Ethanol 2-Butanone | 176 2900 |
| 5. Propane + $NADH_2$ | 1-Propanol 2-Propanol | 15 22 |
| 6. Propane + SADH + $NAD^+$ | 1-Propanol Acetone | 30 85 |
| 7. Propane + 2-Butanol + SADH + $NAD^+$ | 1-Propanol Acetone 2-Butanone | 32 40 2700 |
| 8. Butane + $NADH_2$ | 1-Butanol 2-Butanol | 40 25 |

TABLE XVII (Cont'd)

| Substrate + Cofactor System | Products[a] | Specific Activity[b] (nmoles/min/mg protein) |
|---|---|---|
| 9. Butane + SADH + NAD$^+$ | 1-Butanol | 200 |
| | 2-Butanone | 100 |
| 10. Butane + 2-Butanol + SADH + NAD$^+$ | 1-Butanol | 210 |
| | 2-Butanone | 3000 |
| 11. Pentane + NADH$_2$ | 1-Pentanol | 20 |
| | 2-Pentanol | 45 |
| 12. Pentane + SADH + NAD$^+$ | 1-Pentanol | 80 |
| | 2-Pentanone | 160 |
| 13. Hexane + NADH$_2$ | 1-Hexanol | 39 |
| | 2-Hexanol | 20 |
| 14. Hexane + SADH + NAD$^+$ | 1-Hexanol | 84 |
| | 2-Hexanone | 60 |

(a) Products were identified and estimated by gas chromatography retention time comparison and co-chromatography with authentic standards. Reactions were carried out at 35°C on a shaker bath. Partially purified secondary alcohol dehydrogenase (SADH) was prepared from a yeast, Pichia sp.

(b) Specific activities for epoxidation reactions using NADH$_2$ itself were expressed as the nmoles of product formed/min/mg. of protein in the soluble fractions of Methylobacterium sp. (CRL.26). Specific activities for dehydrogenase reactions were expressed as nmoles of methylketone or 2-butanone formed/min/mg of purified SADH from yeast.

The vials were incubated at 35°C on a reciprocating water bath shaker at 50 oscillations per minute. The gaseous phase of the vials was evacuated by vacuum and replaced with a gaseous mixture consisting of 1:1 by volume oxygen:the alkane substrate indicated in Table XVIII, at which point the reaction was initiated.

The rate of oxidation of the alkane substrates was measured either by estimation of products formed or by amount of substrate utilized from the gas or liquid phase. In the former case, 1-2 $\mu$l samples of the reaction mixture were injected into a gas chromatograph immediately after addition of substrate (zero time) and after 4 and 8 min. of incubation. Specific activities were expressed as nmoles of product formed per min. per mg of protein, with the higher number representing better conversion. If substrate utilization was the measure of oxidation rate, 2 $\mu$l of liquid or 50 $\mu$l of gas was injected into the gas chromatograph at zero time and after 4 and 8 min. of incubation of the reaction mixture at 35°C, and specific activities were expressed as the amount of substrate utilized per min. per mg. of protein. Regardless of the procedure employed, control experiments were conducted for each substrate in the absence of $NADH_2$, in the absence of oxygen, and using boiled extracts.

The alcohol products were identified and estimated by retention time comparisons and co-chromatography with authentic standards using flame-ionization gas chromatography. The column temperature was maintained isothermally between 80°C and 130°C with helium carrier gas flow rates of 20-40 ml per min. The amount of product formed was estimated from peak areas using a standard graph constructed using authentic compounds.

Duplicate measurements were carried out for each substrate. Protein concentrations in cellular fractions were estimated with Folin Ciocalteu reagent as described by O.H. Lowry et al., supra, using bovine serum albumin as a standard.

TABLE XVIII

Hydroxylation of n-Alkanes

| Substrate | Product | Specific Activity (nmoles/min/mg of protein) |
|---|---|---|
| Methane | Methanol | 48 |
| Ethane | Ethanol | 34 |
| Propane | 1-Propanol | 10 |
|  | 2-Propanol | 15 |
| Butane | 1-Butanol | 18 |
|  | 2-Butanol | 12 |
| Pentane | 1-Pentanol | 8 |
|  | 2-Pentanol | 16 |
| Hexane | 1-Hexanol | 14 |
|  | 2-Hexanol | 8 |

EXAMPLE 16 - Oxidation of Substituted Alkane Derivatives

The procedure of Example 15 was followed using as substrates the substituted alkane derivatives in Table XIX. Detection of formaldehyde (the oxidation product of chloromethane and bromomethane) was estimated colorimetrically by the Hantzsch reaction described by T. Nash, Biochem. J., 55, 416 (1953). The results are indicated in Table XIX.

TABLE XIX

Oxidation of Substituted Alkane Deriviatives

| Substrate | Product | Specific Activity (nmoles/min/mg of protein) |
|---|---|---|
| Chloromethane | Formaldehyde | 42 |
| Bromomethane | Formaldehyde | 38 |
| Dichloromethane | ND | 35 |
| Trichloromethane | ND | 25 |
| Nitromethane | ND | 20 |
| 1-Bromobutane | ND | 32 |
| 2-Bromobutane | ND | 16 |
| Isobutane | Isobutanol | 15 |
| | Tert.-butanol | 18 |

ND: Product was not identified.

EXAMPLE 17 - Epoxidation of Alkenes

The procedure of Example 15 was followed to oxidize several alkenes, and the results are shown in Table XX.

## TABLE XX

### Epoxidation of Alkenes

| Substrate | Product | Specific Activity (nmoles/min/mg of protein) |
|---|---|---|
| Ethylene | Ethylene Oxide | 25 |
| Propylene | Propylene Oxide | 50 |
| But-1-ene | 1,2-Epoxybutane | 32 |
| Butadiene | 1,2-Epoxybutene | 30 |
| Isobutylene | 1,2-Epoxyisobutene | 28 |
| But-2-ene | 2,3-Epoxybutane | 18 |
|  | 2-Buten-1-ol | 10 |
| 2-Methyl-1-butene | ND | 21 |
| 2-Methyl-2-butene | ND | 9 |
| 1-Bromo-1-butene | ND | 24 |
| 2-Bromo-2-butene | ND | 8 |
| Isoprene | 1,2-Epoxyisoprene | 16 |

ND: Product was not identified.

It is seen that there are many differences in oxidation rates of alkanes, substituted alkanes, and alkenes when the soluble fraction containing methane monooxygenase from the facultative methylotroph Methylobacterium organophilum (ATCC 27,886) is employed.

EXAMPLE 18 - Oxidation of Ethers and Carbon Monoxide

The procedure of Example 15 was followed to oxidize ethers and carbon monoxide, with the results indicated in Table XXI.

## TABLE XXI

### Oxidation of Ethers

| Substrate | Product | Specific Activity (nmoles/min/mg of protein) |
|---|---|---|
| Dimethylether | Methanol | 20 |
| | Formaldehyde | 10 |
| Butylether | ND | 24 |
| Carbon Monoxide | Carbon Dioxide | 20 |

ND: Product was not identified.

EXAMPLE 19 - Oxidation of Cycloalkyl and Aromatic Compounds

The procedure of Example 15 was followed to oxidize cyclohexane, benzene, and toluene, and the results are indicated in Table XXII.

## TABLE XXII

### Oxidation of Cyclic and Aromatic Compounds

| Substrate | Product | Specific Activity (nmoles/min/mg of protein) |
|---|---|---|
| Cyclohexane | Cyclohexanol | 18 |
| Toluene | Benzylalcohol | 17 |
| Benzene | Phenol | 15 |

EXAMPLE 20 - Regeneration of $NADH_2$ Using $NAD^+$ with Formate as Cofactor System

The procedure of Example 15 was followed using 1.0 ml of a reaction mixture consisting of 50 $\mu$ moles potassium phosphate buffer at pH 7.0, 10 $\mu$ moles $NAD^+$, 10 $\mu$ moles sodium formate, and, as the remainder, the soluble S(80) fraction obtained as described in Example 14. The substrates employed, products produced, and rates of product formation are indicated in Table XXIII.

TABLE XXIII

Regeneration of $NAD^+/NADH_2$:
Oxidation of Alkanes/Alkenes by Soluble
Methane Monooxygenase from <u>Methylobacterium
organophilum</u> (ATCC 27,886) and Oxidation of
Formate by Formate Dehydrogenase

| Substrate | Product | Rate of Product Formation (nmoles/ min/mg of protein) |
|---|---|---|
| Methane | Methanol | 50 |
| Ethane | Ethanol | 35 |
| Propane | Propan-1-ol | 12 |
|  | Propan-2-ol | 18 |
| Ethylene | Ethylene Oxide | 28 |
| Propylene | Propylene Oxide | 54 |
| 1-Butene | 1,2-Epoxybutane | 35 |

The soluble S(80) fraction contains a $NAD^+$-linked formate dehydrogenase with a specific activity of 180 nmoles of $NAD^+$ reduced per min. per mg protein, which was measured spectrophotometrically at 340 nm as described in Patel et al., <u>J. Bacteriol.</u>, <u>136</u>, 352 (1978). In the presence of formate this dehydrogenase enzyme regenerated the cofactor $NADH_2$ required to oxidize alkanes and alkenes, as shown in the above table.

EXAMPLE 21 - Regeneration of $NADH_2$ Using $NAD^+$
with Formaldehyde as Cofactor System

The procedure of Example 15 was followed using 1.0 ml of a reaction mixture consisting of 50 $\mu$ moles potassium phosphate buffer at pH 7.0, 10 $\mu$ moles $NAD^+$, 10 $\mu$ moles reduced glutathione, 10 $\mu$ moles formaldehyde, and, as the remainder, purified formaldehyde dehydro-

genase from yeast <u>Pichia</u> sp. and the soluble S(80) fraction obtained as described in Example 14. The substrates employed, products produced, and rates of product formation are indicated in Table XXIV.

TABLE XXIV

Regeneration of NAD$^+$/NADH$_2$:
Hydroxylation of Alkanes/Epoxidation of Alkenes by Soluble Methane Monooxygenase from <u>Methylobacterium organophilum</u> (ATCC 27,886) and Oxidation of Formaldehyde by Formaldehyde Dehydrogenase

| Substrate | Product | Rate of Product Formation (nmoles/ min/mg of protein) |
|---|---|---|
| Methane | Methanol | 42 |
| Ethane | Ethanol | 30 |
| Butane | Butan-1-ol | 20 |
| | Butan-2-ol | 10 |
| Ethylene | Ethylene Oxide | 28 |
| Propylene | Propylene Oxide | 52 |

EXAMPLE 22 - Regeneration of NADH$_2$ Using NAD$^+$ With Secondary Alcohol Dehydrogenase as Cofactor System

The procedure of Example 15 was followed using 1.0 ml of a reaction mixture consisting of 50 μ moles potassium phosphate buffer at pH 7.0, 10 μ moles NAD$^+$, 10 μ moles secondary alcohol (either 2-butanol or 2-propanol), and, as the remainder, secondary alcohol dehydrogenase from yeast <u>Pichia</u> sp. and the soluble S(80) fraction obtained as described in Example 14. The dehydrogenase was previously purified as described in Patel et al., <u>Eur. J. Biochem.</u>, <u>101</u>, 401 (1979). The substrates employed, products formed and rates of product formation are indicated in Table XXV.

TABLE XXV

Regeneration of $NAD^+/NADH_2$:

Oxidation of Alkanes/Alkenes by Soluble
Methane Monooxygenase from <u>Methylobacterium
organophilum</u> (ATCC 27,886) and Dehydrogenation of
Secondary Alcohol by Secondary Alcohol Dehydrogenase

| Substrates (Alkane or Alkene and Secondary Alcohol) | Respective Products | Rate of Product Formation (nmoles/ min/mg of protein) |
|---|---|---|
| Methane | Methanol | 40 |
| 2-Butanol | 2-Butanone | 50 |
| Ethane | Ethanol | 32 |
| 2-Butanol | 2-Butanone | 48 |
| Propane | Propan-1-ol | 10 |
| 2-Butanol | Acetone | 18 |
| | 2-Butanone | 45 |
| Butane | Butan-1-ol | 15 |
| 2-Propanol | 2-Butanone | 10 |
| | Acetone | 45 |
| Ethylene | Ethylene oxide | 25 |
| 2-Butanol | 2-Butanone | 45 |
| Propylene | Propylene oxide | 48 |
| 2-Butanol | 2-Butanone | 50 |
| Isobutylene | Epoxyisobutylene | 29 |
| 2-Propanol | Acetone | 45 |

EXAMPLE 23 – Regeneration Using Other Cofactor Systems

When $NADH_2$ replaced $NADH_2$ as electron donor in the procedure of Example 15, about 70% of the soluble methane monooxygenase activity was observed. Ascorbate or methanol in the presence of methanol dehydrogenase, however, could not replace $NADH_2$ as an electron donor.

The soluble methane monooxygenase fractions from the obligate methane-utilizing bacteria, <u>Methylo-coccus capsulatus</u> Strain Bath and <u>Methylosinus tricho-sporium</u> OB3b, were disclosed for use in oxidation processes in U.K. Pat. 1,603,864. The soluble mono-oxygenase fraction from facultative methylotrophic organisms such as Methylobacterium organophilum (ATCC 27,886) and the newly discovered facultative strain Methylobacterium organophilum (CRL.26) has now been isolated and is discovered as capable of oxidizing an oxidizable organic substrate under aerobic conditions in the presence of a cofactor system of $NADH_2$ or $NADPH_2$. The fraction of the CRL.26 strain is further found to have different oxidation rates from those of the soluble fraction of Methylococcus capsulatus .Strain Bath for a given substrate (e.g., higher conversion rates of $C_6-C_8$ alkanes) and to behave differently with respect to potential electron donors (cofactors) and inhibitors of activity.

CLAIMS:

1. A soluble fraction of a facultative organism or genetically engineered derivative thereof or natural mutant thereof grown on a $C_1$ compound, which fraction is characterised as being capable of oxidizing organic compounds in the presence of a cofactor system comprising $NADH_2$ or $NADPH_2$.

2. A soluble fraction according to claim 1 wherein the organism is of the Methylobacterium genus..

3. A soluble fraction according to claims 1 or 2 wherein the organism is of the Methylobacterium organophilum species.

4. A soluble fraction according to any one of claims 1 to 3 wherein the organism is of the strain Methylobacterium organophilum (CRL.26) (NRRL B-11,222) or Methylobacterium organophilum (ATCC 27,886).

5. A soluble fraction according to any one of claims 1 to 4 wherein the $C_1$ compound is methane.

6. A soluble fraction according to any one of claims 1 to 5 wherein the organism is aerobically grown in a fermenter under continuous gassing with a mixture of a $C_1$ compound and air.

7. A process for increasing the oxidative state of an oxidizable organic compound which comprises oxidizing the oxidizable substrate, under aerobic conditions, in the presence of a soluble fraction according to any one of claims 1 to 6 and a cofactor

system comprising $NADH_2$ or $NADPH_2$, until at least a portion of the corresponding oxidized product is produced in isolatable amounts.

8. A process according to claim 7 wherein the $C_1$ compound is methane and the organic compound is an alkene, an alkane, an ether, benzene, toluene or carbon monoxide.

9. A process according to claim 7 or 8 wherein the $NADH_2$ or $NADPH_2$ is added exogenously to the oxidation reaction mixture.

10. A process according to claim 7 or 8 wherein the $NADH_2$ is generated or regenerated in situ.